# EUROPEAN PATENT APPLICATION

(11) **EP 4 497 450 A1**
(43) Date of publication of application: **29.01.2025**
(21) Application number: 23829410.2
(22) Date of filing: 16.02.2023
(51) Int. Cl.: A61L 27/36, A61L 27/50

(54) **TREATMENT METHOD FOR BIOLOGICAL TISSUE AND MEDICAL MATERIAL**

(30) Priority: 28.06.2022 CN 202210752185
(71) Applicant: Jiangsu Trulive Medtech Co., Ltd., Nantong, Jiangsu 226400 (CN)
(72) Inventor: YANG, Wei, Nantong, Jiangsu 226400 (CN); WEI, Yongqiang, Nantong, Jiangsu 226400 (CN)
(74) Representative: Pohlman, Sandra M.
(86) International application number: PCT/CN2023/076496
(87) International publication number: WO 2024/001217

(57) **Abstract**

The present invention provides a treatment method and a medical material for biological tissue. The method includes the steps of: subjecting the biological tissue to a cross-linking process, wherein the biological tissue from the cross-linking process has active groups on its surface; subjecting the biological tissue from the cross-linking process to an end-capping process so that at least some of the active groups are end-capped; and subjecting the biological tissue from the end-capping process to an anti-oxidation and sterilization process. The medical material is obtained by treating biological tissue according to the method. It has fewer active groups on the surface and therefore forms fewer calcification binding sites when implanted into a patient's body. Therefore, the medical material exhibits higher resistance to calcification.

## Description

### TECHNICAL FIELD

The present invention relates to the field of medical devices, and particularly to a treatment method and a medical material for biological tissue.

### BACKGROUND

As the economy is developing and people's living standards are improving, biomedical materials have been widely valued and used as one of the effective tools for humans to fight disease. Specifically, biomedical materials can be used to manufacture bone, tooth, joint, tendon and other musculoskeletal prostheses, skin, esophagus, respiratory tract, bladder and other soft tissue prostheses, prosthetic heart valves and blood vessels, cardiovascular catheters and other cardiovascular prostheses, and membranes for medical use, such as membranes for blood purification and separation, selectively gas-permeable membranes, contact lenses, etc. As the basis for studies on artificial organs and medical devices, today, biomedical materials have become an important discipline of materials science.

Biomedical materials can be categorized by composition and properties into biomedical metal materials, biomedical inorganic materials, biomedical polymeric materials, biomedical composite materials and biologically-derived materials. Biologically-derived materials, also known as bioregenerative materials, are formed by specially treating natural biological tissue from allogeneic or xenogeneic animals. The special treatment may be moderate and preserves the native structure of biological tissue, such as fixation, sterilization or eliminate antigenicity. It may also be an aggressive treatment, which disrupts the native structure of biological tissue and transforms it into a new physical form. As biological tissue loses vitality after being so treated, the resulting biologically-derived materials are inanimate. However, as they remain similar to the original native tissue in terms of structure, function or composition, these materials can play an important role in the repair and replacement of human tissue.

At present, biologically-derived materials commonly used in the manufacture of artificial heart valves, biological patches and the like are derived from collagenous fibrous tissue of animal origins. However, such products are prone to calcification after implantation. Consequently, the materials may harden or rupture and eventually fail. Many of these materials are stored in glutaraldehyde and/or formaldehyde and treated with a chemical reagent for cross-linking and fixation. A large number of studies have shown that active groups, such as amino groups, aldehyde groups and carboxyl groups (resulting from oxidation of aldehyde groups) that remain on cross-linked tissue may become calcification binding sites and cause calcification after the biological tissue is implanted into a human body.

### SUMMARY

It is an objective of the present invention to provide a treatment method and a medical material for biological tissue. The biological tissue treated using the method exhibits increased resistance to calcification.

The above objective is attained by a method of treating biological tissue provided in the present invention, which includes the steps of:
subjecting the biological tissue to a cross-linking process, wherein the biological tissue from the cross-linking process has active groups on its surface;
subjecting the biological tissue from the cross-linking process to an end-capping process so that at least some of the active groups are end-capped; and
subjecting the biological tissue from the end-capping process to an anti-oxidation and sterilization process.

Optionally, a cross-linking agent used may include glutaraldehyde, wherein the active groups include amino groups, free aldehyde groups and free carboxyl groups.

Optionally, the method may further include:
after the biological tissue is subjected to the cross-linking process and before the biological tissue from the cross-linking process is subjected to the end-capping process,
subjecting the biological tissue to a first cleaning process.

Optionally, a cleaning agent used in the first cleaning process may include any of a physiological saline solution, a phosphate buffer with a pH of 6.8 to 8.6, a D-Hanks solution with a pH of 6.8 to 8.6 or a glutaraldehyde solution with a weight percentage of glutaraldehyde of 0.1% to 2% and a pH of 6.8 to 8.6.

Optionally, the end-capping step may include:
subjecting the biological tissue from the cross-linking process to a first end-capping process using a first end-capping solution to end-cap at least some of the amino groups;
subjecting the biological tissue from the first end-capping process to a second end-capping process using a second end-capping solution to end-cap at least some of the free aldehyde groups; and
subjecting the biological tissue from the second end-capping process to a third end-capping process using a third end-capping solution to end-cap at least some of the free carboxyl groups.

Optionally, the first end-capping solution may include a first end-capping agent including at least one of heparin, mannose, formaldehyde and galactose.

Additionally or alternatively, the second end-capping solution may include a second end-capping agent including at least one of sodium borohydride, taurine, polyvinyl alcohol and an aminosulfate.

Additionally or alternatively, the third end-capping solution may include a third end-capping agent including at least one of N-hydroxysulfosuccinimide, 1-(3-dimethylaminopropyl)-3-ethylcarbodiimide hydrochloride and an amino compound.

Optionally, the first end-capping solution may have a pH of 5.0 to 9.0, wherein any component of the first end-capping agent is present in the first end-capping solution at a weight percentage of 0.01% to 10% and the first end-capping process is performed at a temperature of 0 to 50 °C for 1 h to 72 h.

Additionally, the second end-capping solution may have a pH of 5.0 to 9.0, wherein any component of the second end-capping agent is present in the second end-capping solution at a weight percentage of 0.01% to 10% and the second end-capping process is performed at a temperature of 0 to 50 °C for 1 h to 72 h.

Additionally, the third end-capping solution may have a pH of 5.0 to 9.0, wherein any component of the third end-capping agent is present in the third end-capping solution at a weight percentage of 0.01% to 10% and the third end-capping process is performed at a temperature of 0 to 50 °C for 1 h to 72 h.

Optionally, the end-capping step may further include: after the first end-capping process and prior to the second end-capping process, subjecting the biological tissue from the first end-capping process to a second cleaning process;
after the second end-capping process and prior to the third end-capping process, subjecting the biological tissue from the second end-capping process to a third cleaning process; and
after the third end-capping process and before the biological tissue from the end-capping process is subjected to the anti-oxidation and sterilization process, subjecting the biological tissue to a fourth cleaning process.

Optionally, the second cleaning process may use a glutaraldehyde solution as a cleaning agent, which contains glutaraldehyde at a weight percentage of 0.01% to 2% and has a pH of 6.8 to 8.6.

Additionally or alternatively, the third cleaning process may use the second end-capping agent as a cleaning agent.

Additionally or alternatively, the fourth cleaning process may use the third end-capping agent as a cleaning agent.

Optionally, the anti-oxidation and sterilization process may be performed on the biological tissue from the end-capping process using a combined solution of a sterilizing agent and an antioxidant, the sterilizing agent including any of glutaraldehyde and formaldehyde, the antioxidant including at least one of vitamin C, vitamin E and a reducing sugar, the antioxidant present in the combined solution at a weight percentage of 0.01% to 10%, the sterilizing agent present in the combined solution at a weight percentage of 0.01% to 10%, the combined solution having a pH of 6.8 to 8.6.

Optionally, the anti-oxidation and sterilization process may be performed at a temperature of 0 to 50 °C for 1 h to 72 h.

Optionally, the biological tissue may include any of a pericardium, heart valve, pulmonary pleuron, small intestinal submucosa, cranial dura mater, spinal dura mater, ligament and animal skin.

The above objective is also attained by a medical material provided in the present invention, which is obtained by treating biological tissue according to the method as defined in any of the above paragraphs.

Compared with the prior art, the method and medical material of the present invention have the advantages as follows:
The method includes the steps of: subjecting biological tissue to a cross-linking process, wherein the biological tissue from the cross-linking process has active groups on its surface; subjecting the biological tissue from the cross-linking process to an end-capping process so that at least some of the active groups are end-capped; and subjecting the biological tissue from the end-capping process to an anti-oxidation and sterilization process. The end-capping process end-caps and thereby deactivates the active groups, making them unable to form calcification binding sites. Therefore, after the treated biological tissue is implanted into a human body, it exhibits higher resistance to calcification.

### BRIEF DESCRIPTION OF THE DRAWINGS

The accompanying drawings are provided to facilitate a better understanding of the present invention and do not unduly limit the scope thereof in any sense, in which:
Fig. 1 compares the results of tests conducted in Example 1 and Comparative Examples 1, 2 and 3 in accordance with the present invention.
Fig. 2 is a schematic diagram comparing presence of calcium on medical materials obtained in Example 2 and Comparative Examples 4, 5 and 6 in accordance with the present invention 8 weeks after the medical material were subcutaneously implanted into rats.
Fig. 3 is a schematic diagram comparing presence of calcium on medical materials obtained in Example 3 and Comparative Examples 7, 8 and 9 in accordance with the present invention 8 weeks after the medical material were subcutaneously implanted into rats.
Fig. 4 is a schematic diagram comparing presence of calcium on medical materials obtained in Example 4 and Comparative Examples 10, 11 and 12 in accordance with the present invention 8 weeks after the medical material were subcutaneously implanted into rats.
Fig. 5 is a schematic diagram comparing presence of calcium on medical materials obtained in Example 5 and Comparative Examples 13, 14 and 15 in accordance with the present invention 8 weeks after the medical material were subcutaneously implanted into rats.
Fig. 6 is a schematic diagram comparing presence of calcium on medical materials obtained in Example 6 and Comparative Examples 16, 17 and 18 in accordance with the present invention 8 weeks after the medical material were subcutaneously implanted into rats.

### DETAILED DESCRIPTION

Embodiments of the present invention will be described below by way of specific examples. Based on the disclosure and teachings provided herein, a person of ordinary skill in the art will readily realize other advantages and benefits provided by the present invention. The present invention may also be otherwise embodied or applied through different embodiments, and various modifications or changes may be made to the details disclosed herein from different points of view or for different applications, without departing from the spirit of the present invention. It should be noted that the accompanying drawings are provided herein merely to schematically illustrate the basic concept of the present invention. Accordingly, they only show components relating to the present invention but not necessarily depict all the components as well as their real shapes and dimensions in practical implementations. In practice, the configurations, counts and relative scales of the components may vary arbitrarily and their arrangements may be more complicated.

In the following, each of the embodiments is described as having one or more technical features. However, this does not mean that the present invention must be practiced necessarily with all such technical features, or separately with some or all the technical features in any of the embodiments. In other words, as long as the present invention can be put into practice, a person skilled in the art may choose some or all of the technical features in any of the embodiments or combine some or all of the technical features in different embodiments based on the teachings herein and depending on relevant design specifications or the requirements of practical applications. In this way, the present invention can be carried out more flexibly.

As used herein, the singular forms "a", "an" and "the" include plural referents, and the plural form "a plurality of" means "two or more", unless the context clearly dictates otherwise. As used herein, the term "or" is generally employed in the sense including "and/or", unless the context clearly dictates otherwise. Those of ordinary skill in the art can understand the specific meanings of the above-mentioned terms herein, depending on their context.

It is an objective of the present invention to provide a method of treating biological tissue obtained from an animal. The treated biological tissue is obtained as a biologically-derived material usable in the medical field. Without limitation, the biological tissue may include a pericardium, heart valve, peritonaeum, pulmonary pleuron, small intestinal submucosa, cranial dura mater, spinal dura mater, ligament, skin or the like, which is obtained from the animal. The animal may be a bovine animal, pig, rabbit or the like.

The treatment method includes the steps as follows:
Step S10: Subject the biological tissue to a cross-linking process. The biological tissue from the cross-linking process has active groups on its surface.
Step S20: Subject the biological tissue from the cross-linking process to an end-capping process so that at least some of the active groups are end-capped.
Step S30: Subject the biological tissue from the end-capping process to an anti-oxidation and sterilization process, thereby forming a biologically-derived material.

Subjecting the biological tissue from the cross-linking process further to the end-capping process can deactivate at least some of the active groups remaining on the surface of the biological tissue. After the biologically-derived material is implanted into a patient's body, the deactivated active groups cannot bind with calcium, i.e., they do not form calcification sites. As a result, calcification of the biologically-derived material can be mitigated or even eliminated. That is, the biologically-derived material is more resistant to calcification.

Optionally, in step S10, the biological tissue may be cross-linked with an aldehyde compound. In a non-limiting example, the biological tissue is cross-linked with a glutaraldehyde solution. Those skilled in the art will appreciate that the biological tissue contains large amounts of various proteins, the various proteins are composed of amino acids, and when using a glutaraldehyde solution to cross-link the biological tissue, a nucleophilic addition-dehydration reaction will occur between aldehyde groups in glutaraldehyde and amino groups in the amino acids, forming a Schiff base containing C=N groups. As each glutaraldehyde molecule has one aldehyde group at each end, it is possible that after the process, there are some glutaraldehyde molecules with the aldehyde group at one end being cross-linked to an amino group and the aldehyde group at the other end remaining free. Moreover, such cross-linking tends to be insufficient, and not all amino groups on the biological tissue can combine with an aldehyde group, leaving unreacted free amino groups on the biological tissue. Further, in the cross-linking process, there might also be some free aldehyde groups oxidized into free carboxyl groups. Therefore, the active groups remaining on the surface of the biological tissue from the cross-linking process may at least include amino groups, free aldehyde groups and free carboxyl groups. If this biological tissue from the cross-linking process is directly implanted into a patient's body, the amino groups, free aldehyde groups and free carboxyl groups on the surface of the biological tissue will form calcification binding sites, leading to proneness of the biological tissue to calcification, which is detrimental to its service life.

Therefore, after step S10, the treatment method further includes the step S20, the amino groups, free aldehyde groups and free carboxyl groups remaining on the surface of the biological tissue from the cross-linking process are end-capped so that at least some of the active groups are deactivated. In step S20, the amino groups, free aldehyde groups and free carboxyl groups may be successively end-capped in an appropriate temporal order. Accordingly, step S20 may further include steps S21, S22 and S23, as described below. In step S21, the biological tissue from the cross-linking process is subjected to a first end-capping process using a first end-capping solution so that at least some of the amino groups are end-capped. In step S22, the biological tissue from the first end-capping process is subjected to a second end-capping process using a second end-capping solution so that at least some of the free aldehyde groups are end-capped. In step S23, the biological tissue from the second end-capping process is subjected to a third end-capping process using a third end-capping solution so that at least some of the free carboxyl groups are end-capped.

The first end-capping solution may include a first end-capping agent, which may include, but is not limited to including, at least one of heparin, mannose, formaldehyde and galactose. That is, the first end-capping agent may have one, two or more components. The first end-capping solution may have a pH of 5.0 to 9.0, and any component of the first end-capping agent may be present in the first end-capping solution at a weight percentage of 0.01% to 10%. The first end-capping process may be performed at a temperature of 0 to 50 °C for 1 h to 72 h. The second end-capping solution may include a second end-capping agent, which may include at least one of sodium borohydride, taurine, polyvinyl alcohol and an aminosulfate. That is, the second end-capping agent may have one, two or more components. The second end-capping solution may have a pH of 5.0 to 9.0, and any component of the second end-capping agent may be present in the first end-capping solution at a weight percentage of 0.01% to 10%. The second end-capping process may be performed at a temperature of 0 to 50 °C for 1 h to 72 h. The third end-capping solution may include a third end-capping agent, which may include at least one of N-hydroxysulfosuccinimide, 1-(3-dimethylaminopropyl)-3-ethylcarbodiimide hydrochloride and an amino compound. That is, the third end-capping agent may have one, two or more components. The third end-capping solution may have a pH of 5.0 to 9.0, and any component of the third end-capping agent may be present in the third end-capping solution at a weight percentage of 0.01% to 10%. The third end-capping process may be performed at a temperature of 0 to 50 °C for 1 h to 72 h. Note that, any pH as mentioned herein is measured at room temperature.

The method may further include a first cleaning step. Specifically, after step S10 is completed and before step S21 is performed, the biological tissue from the cross-linking process may be subjected to a first cleaning process.

Additionally, the multiple cleaning processes may be performed in the end-capping step. Specifically, after step S21 is completed and before step S22 is performed, the biological tissue from the first end-capping process may be subjected to a second cleaning process. After step S22 is completed and before step S23 is performed, the biological tissue from the second end-capping process may be subjected to a third cleaning process. After step S23 is completed and before step S30 is performed, the biological tissue from the third end-capping process may be subjected to a fourth cleaning process.

Optionally, a cleaning agent used in the first cleaning process may include any of a physiological saline solution, a phosphate buffer with a pH of 6.8 to 8.6, a D-Hanks solution with a pH of 6.8 to 8.6 or a glutaraldehyde solution with a weight percentage of glutaraldehyde of 0.1% to 2% and a pH of 6.8 to 8.6. Typically, the first cleaning process is carried out at room temperature and involves 3 to 5 cleaning cycles, each lasting for 3 to 5 min. Using a glutaraldehyde solution as a cleaning agent in the first cleaning process is advantageous in that it effects supplementary cross-linking of the biological tissue from the cross-linking process, allowing the amino groups remaining on the biological tissue to combine with additional aldehyde groups in the glutaraldehyde and thereby be eliminated.

A cleaning agent used in the second cleaning process may be a glutaraldehyde solution with a glutaraldehyde concentration of 0.01% to 2% and a pH of 6.8 to 8.6. The second cleaning process may be carried out at room temperature and involve 3 to 5 cleaning cycles, each lasting for 3 to 5 min. This cleaning process is performed to allow the glutaraldehyde used therein to additionally react and combine with the amino groups remaining on the biological tissue from the first end-capping process, forming free aldehyde groups. At least some of these resulting free aldehyde groups will be end-capped in the second end-capping process.

The third cleaning process may use the second end-capping agent as a cleaning agent. This cleaning process may be carried out at room temperature and involve 3 to 5 cleaning cycles, each lasting for 3 to 5 min. This cleaning process is performed for further end-capping of the free aldehyde groups. It is to be noted that, during the second end-capping process and the third cleaning process, at least some of the free aldehyde groups are oxidized into free carboxyl groups.

The fourth cleaning process may use the third end-capping agent as a cleaning agent. This cleaning process may be carried out at room temperature and involve 3 to 5 cleaning cycles, each lasting for 3 to 5 min. This cleaning process is performed for further end-capping of at least some of the free carboxyl groups.

Further, in step S30, a combined solution of a sterilizing agent and an antioxidant may be used to perform the anti-oxidation and sterilization process on the biological tissue from the end-capping process. Optionally, the sterilizing agent may include, but is not limited to including, any of glutaraldehyde and formaldehyde. Optionally, the antioxidant may include, but is not limited to including, at least one of vitamin C, vitamin E and a reducing sugar. Those skilled in the art will appreciate that the reducing sugar may include, but is not limited to including, at least one of glucose, fructose, maltose and galactose. The combined solution may have a pH of 6.8 to 8.6. Moreover, the antioxidant may be present in the combined solution at a weight percentage of 0.01% to 10%, and the sterilizing agent may be present in the combined solution at a weight percentage of 0.01% to 10%. The anti-oxidation and sterilization process may be carried out at a temperature of 0 to 50 °C for 1 h to 72 h. Aldehyde groups remaining in the biologically-derived material from the anti-oxidation and sterilization process will not be oxidized to form new calcification binding sites, making the biologically-derived material more resistant to calcification.

In embodiments of the present invention, there is also provided a biologically-derived material which can be obtained by treating biological tissue according to the method as described above.

Objectives, advantages and features of the present invention will become more apparent from the following detailed description of examples of embodiment thereof. Note that the figures are provided in a very simplified form not necessarily drawn to exact scale for the only purpose of helping to explain the disclosed embodiments in a more convenient and clearer way. Throughout these drawings, like numerals indicate like elements.

### <Example 1>

In Example 1, a bovine pericardium was treated as biological tissue as follows.

First of all, the bovine pericardium was freshly obtained from a slaughterhouse. After undergoing stripping, selection and cleaning processes, it was subjected to a cross-linking process carried out for 14 days using a glutaraldehyde solution containing glutaraldehyde at a weight percentage of 0.625%. The bovine pericardium from the cross-linking process was then cut (on a cutter, model Trotec Speed x-100) into a 50 mm × 50 mm sample.

After that, a first cleaning process was performed on the sample at room temperature using a glutaraldehyde solution containing glutaraldehyde at a weight percentage of 0.625% and having a pH of 7.4. In this process, the sample was cleaned three times, 3 min each time.

Next, the sample from the first cleaning process was subjected to a first end-capping process carried out at room temperature for 24 h using a first end-capping solution. The first end-capping solution has a pH of 7.4 and contained heparin as a first end-capping agent at a weight percentage of 0.5%. It is to be noted that, as used herein, the term "room temperature" refers to an indoor temperature generally in the range of 20 °C ± 5 °C.

Subsequently, the sample from the first end-capping process was subjected to a second cleaning process carried out at room temperature. In the second cleaning process, a glutaraldehyde solution containing glutaraldehyde at a weight percentage of 0.625% and having a pH of 7.4 was used as a cleaning agent, and the sample was cleaned three times, 5 min each time.

Afterwards, the sample from the second cleaning process was subjected to a second end-capping process carried out at 5 °C for 72 h using a second end-capping solution. The second end-capping solution had a pH of 5.0 and contained sodium borohydride as a second end-capping agent at a weight percentage of 1.5%.

A third cleaning process was then performed at room temperature on the sample from the second end-capping process. In the third cleaning process, the second end-capping solution was used as a cleaning agent, and the sample was cleaned three times, 5 min each time.

After that, a third end-capping solution was used to perform a third end-capping process at room temperature on the sample from the third cleaning process. The process was carried out for 72 h, and the third end-capping solution has a pH of 5.0 and contained 1-(3-dimethylaminopropyl)-3-ethylcarbodiimide hydrochloride (EDC) as a third end-capping agent at a weight percentage of 3.4%.

Afterwards, the sample from the third end-capping process was subjected to a fourth cleaning process performed at room temperature using the third end-capping solution as a cleaning agent. In the process, the sample was cleaned three times, 3 min each time.

Finally, a combined solution of an antioxidant and a sterilizing agent was used to perform an anti-oxidation and sterilization process on the sample from the fourth cleaning process at 5 °C, obtaining a biologically-derived material. The anti-oxidation and sterilization process was carried out for 72 h, and the combined solution has a pH of 7.4. The sterilizing agent was glutaraldehyde and present at a weight percentage of 0.625%, and the antioxidant was vitamin C and present at a weight percentage of 0.5%.

### <Comparative Example 1>

Comparative Example 1 differs from Example 1 in that a bovine pericardium was subjected only to the cross-linking process and the first cleaning process but not to the three end-capping processes and the second, third and fourth cleaning processes.

### <Comparative Example 2>

Comparative Example 2 differs from Example 1 in that a bovine pericardium was subjected only to the cross-linking process, the first cleaning process, the first end-capping process and the second cleaning process but not to the second end-capping process, the third cleaning process, the third end-capping process, the fourth cleaning process and the anti-oxidation and sterilization process.

### <Comparative Example 3>

Comparative Example 3 differs from Example 1 in that a bovine pericardium was subjected only to the cross-linking process, the first cleaning process, the first end-capping process, the second cleaning process, the second end-capping process and the third cleaning process but not to the third end-capping process, the fourth cleaning process and the anti-oxidation and sterilization process.

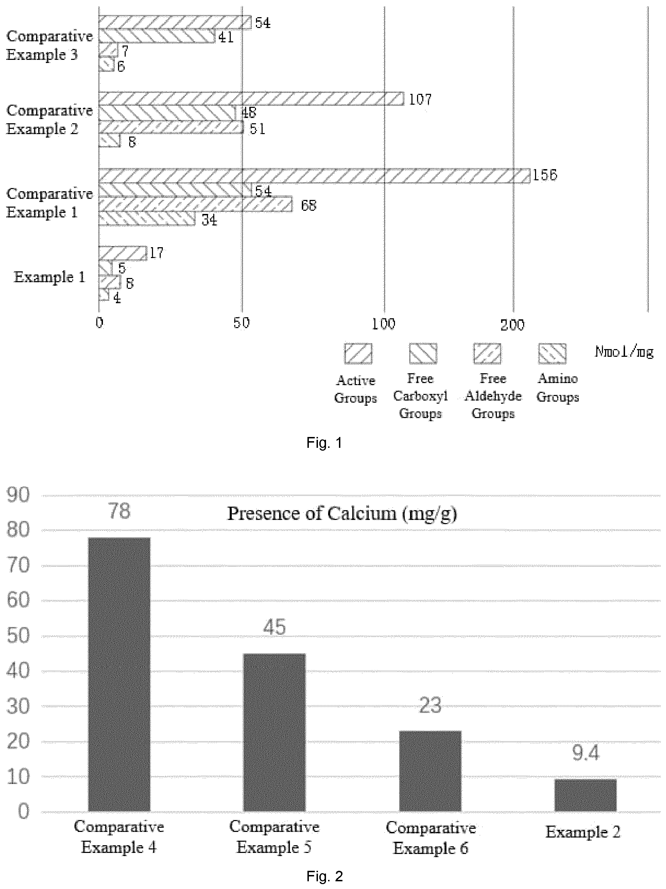

Presence of amino groups, free aldehyde groups and free carboxyl groups on the surface of the biologically-derived materials from Example 1 and Comparative Examples 1, 2 and 3 was measured using spectrophotometry, and the results are presented in Fig. 1. As can be seen from Fig. 1, active groups were present on the surface of the biologically-derived material from Comparative Example 1 at 156 nmol/ml, including 34 nmol/mg of amino groups, 68 nmol/mg of free aldehyde groups and 54 nmol/mg of free carboxyl groups. Active groups were present on the surface of the biologically-derived material from Comparative Example 2 at 107 nmol/mg, including 8 nmol/mg of amino groups, 51 nmol/mg of free aldehyde groups and 48 nmol/mg of free carboxyl groups. Active groups were present on the surface of the biologically-derived material from Comparative Example 3 at 54 nmol/mg, including 6 nmol/mg of amino groups, 7 nmol/mg of free aldehyde groups and 41 nmol/mg of free carboxyl groups. Active groups were present on the surface of the biologically-derived material from Example 1 at 17 nmol/mg, including 4 nmol/mg of amino groups, 8 nmol/mg of free aldehyde groups and 5 nmol/mg of free carboxyl groups. Therefore, the first end-capping process and the second cleaning process can greatly reduce amino groups on the surface of the biological tissue. The second end-capping process and the third cleaning process can greatly reduce free aldehyde groups on the surface of the biological tissue. The three end-capping processes, the four cleaning processes and the anti-oxidation and sterilization process can greatly reduce amino groups, free aldehyde groups and free carboxyl groups on the surface of the biological tissue.

### <Example 2>

In Example 2, a bovine pericardium was treated as biological tissue as follows.

First of all, the bovine pericardium was freshly obtained from a slaughterhouse. After undergoing stripping, selection and cleaning processes, it was subjected to a cross-linking process carried out for 14 days using a glutaraldehyde solution containing glutaraldehyde at a weight percentage of 0.625%. The bovine pericardium from the cross-linking process was then cut (on a cutter, model Trotec Speed x-100) into a 50 mm × 50 mm sample.

After that, a first cleaning process was performed on the sample at room temperature using a phosphate buffer solution with pH of 7.4. In this process, the sample was cleaned five times, 5 min each time.

Next, the sample from the first cleaning process was subjected to a first end-capping process carried out at room temperature for 48 h using a first end-capping solution. The first end-capping solution has a pH of 6.2 and contained formaldehyde as a first end-capping agent at a weight percentage of 2%.

Subsequently, the sample from the first end-capping process was subjected to a second cleaning process carried out at room temperature. In the second cleaning process, a glutaraldehyde solution containing glutaraldehyde at a weight percentage of 0.625% and having a pH of 7.4 was used as a cleaning agent, and the sample was cleaned three times, 3 min each time.

Afterwards, the sample from the second cleaning process was subjected to a second end-capping process carried out at 39 °C for 12 h using a second end-capping solution. The second end-capping solution had a pH of 7.4 and contained taurine as a second end-capping agent at a weight percentage of 0.5%.

A third cleaning process was then performed at room temperature on the sample from the second end-capping process. In the third cleaning process, the second end-capping solution was used as a cleaning agent, and the sample was cleaned three times, 5 min each time.

After that, a third end-capping solution was used to perform a third end-capping process at room temperature on the sample from the third cleaning process. The process was carried out for 72 h, and the third end-capping solution has a pH of 8.6 and contained N-hydroxysulfosuccinimide as a third end-capping agent at a weight percentage of 4.5%.

Afterwards, the sample from the third end-capping process was subjected to a fourth cleaning process performed at room temperature using the third end-capping solution as a cleaning agent. In the process, the sample was cleaned three times, 3 min each time.

Finally, a combined solution of an antioxidant and a sterilizing agent was used to perform an anti-oxidation and sterilization process on the sample from the fourth cleaning process at 45 °C, obtaining a biologically-derived material. The anti-oxidation and sterilization process was carried out for 10 h, and the combined solution has a pH of 8.3. The sterilizing agent was glutaraldehyde and present at a weight percentage of 2.0%, and the antioxidant was vitamin C and present at a weight percentage of 8.5%.

### <Comparative Example 4>

Comparative Example 4 differs from Example 2 in that a bovine pericardium was subjected only to the cross-linking process and the first cleaning process but not to the three end-capping processes and the second, third and fourth cleaning processes.

### <Comparative Example 5>

Comparative Example 5 differs from Example 2 in that a bovine pericardium was subjected only to the cross-linking process, the first cleaning process, the first end-capping process and the second cleaning process but not to the second end-capping process, the third cleaning process, the third end-capping process, the fourth cleaning process and the anti-oxidation and sterilization process.

### <Comparative Example 6>

Comparative Example 6 differs from Example 2 in that a bovine pericardium was subjected only to the cross-linking process, the first cleaning process, the first end-capping process, the second cleaning process, the second end-capping process and the third cleaning process but not to the third end-capping process, the fourth cleaning process and the anti-oxidation and sterilization process.

The biologically-derived materials from Example 2 and Comparative Examples 4, 5 and 6 were separately subcutaneously implanted into rats and taken out 8 weeks later. Presence of calcium on the biologically-derived materials was then measured using atomic absorption spectrophotometry, and the results are presented in Fig. 2. As can be seen from Fig. 2, the biologically-derived material obtained without end-capping (i.e., the biologically-derived material from Comparative Example 4) showed the greatest presence of calcium, and the biologically-derived material obtained by subjecting the tissue to only the first end-capping process (i.e., the biologically-derived material from Comparative Example 5) showed the second greatest presence of calcium. Moreover, the biologically-derived material obtained by subjecting the tissue to only the first and second end-capping processes (i.e., the biologically-derived material from Comparative Example 6) showed the third greatest presence of calcium, and the biologically-derived material obtained by subjecting the tissue to all the three end-capping processes and the anti-oxidation and sterilization process (i.e., the biologically-derived material from Example 2) showed the least presence of calcium, i.e., the biologically-derived material obtained by subjecting the tissue to all the three end-capping processes and the anti-oxidation and sterilization process has the best resistance to calcification.

### <Example 3>

In Example 3, a bovine pericardium was treated as biological tissue as follows.

First of all, the bovine pericardium was freshly obtained from a slaughterhouse. After undergoing stripping, selection and cleaning processes, it was subjected to a cross-linking process carried out for 14 days using a glutaraldehyde solution containing glutaraldehyde at a weight percentage of 0.625%. The bovine pericardium from the cross-linking process was then cut (on a cutter, model Trotec Speed x-100) into a 50 mm × 50 mm sample.

After that, a first cleaning process was performed on the sample at room temperature using a phosphate buffer solution with pH of 7.4. In this process, the sample was cleaned five times, 5 min each time.

Next, the sample from the first cleaning process was subjected to a first end-capping process carried out at 48 °C for 6 h using a first end-capping solution. The first end-capping solution has a pH of 8.6 and contained mannose as a first end-capping agent at a weight percentage of 1%.

Subsequently, the sample from the first end-capping process was subjected to a second cleaning process carried out at room temperature. In the second cleaning process, a glutaraldehyde solution containing glutaraldehyde at a weight percentage of 0.625% and having a pH of 7.4 was used as a cleaning agent, and the sample was cleaned three times, 3 min each time.

Afterwards, the sample from the second cleaning process was subjected to a second end-capping process carried out at 50 °C for 72 h using a second end-capping solution. The second end-capping solution had a pH of 5.0 and contained polyvinyl alcohol as a second end-capping agent at a weight percentage of 1.2%.

A third cleaning process was then performed at room temperature on the sample from the second end-capping process. In the third cleaning process, the second end-capping solution was used as a cleaning agent, and the sample was cleaned three times, 5 min each time.

After that, a third end-capping solution was used to perform a third end-capping process at room temperature on the sample from the third cleaning process. The process was carried out for 72 h, and the third end-capping solution has a pH of 8.0 and contained 2-hydroxyethylamine as a third end-capping agent at a weight percentage of 0.5%.

Afterwards, the sample from the third end-capping process was subjected to a fourth cleaning process performed at room temperature using the third end-capping solution as a cleaning agent. In the process, the sample was cleaned three times, 3 min each time.

Finally, a combined solution of an antioxidant and a sterilizing agent was used to perform an anti-oxidation and sterilization process on the sample from the fourth cleaning process at 5 °C, obtaining a biologically-derived material. The anti-oxidation and sterilization process was carried out for 72 h, and the combined solution has a pH of 6.8. The sterilizing agent was glutaraldehyde and present at a weight percentage of 0.625%, and the antioxidant was vitamin E and present at a weight percentage of 2.3%.

### <Comparative Example 7>

Comparative Example 7 differs from Example 3 in that a bovine pericardium was subjected only to the cross-linking process and the first cleaning process but not to the three end-capping processes and the second, third and fourth cleaning processes.

### <Comparative Example 8>

Comparative Example 8 differs from Example 3 in that a bovine pericardium was subjected only to the cross-linking process, the first cleaning process, the first end-capping process and the second cleaning process but not to the second end-capping process, the third cleaning process, the third end-capping process, the fourth cleaning process and the anti-oxidation and sterilization process.

### <Comparative Example 9>

Comparative Example 9 differs from Example 3 in that a bovine pericardium was subjected only to the cross-linking process, the first cleaning process, the first end-capping process, the second cleaning process, the second end-capping process and the third cleaning process but not to the third end-capping process, the fourth cleaning process and the anti-oxidation and sterilization process.

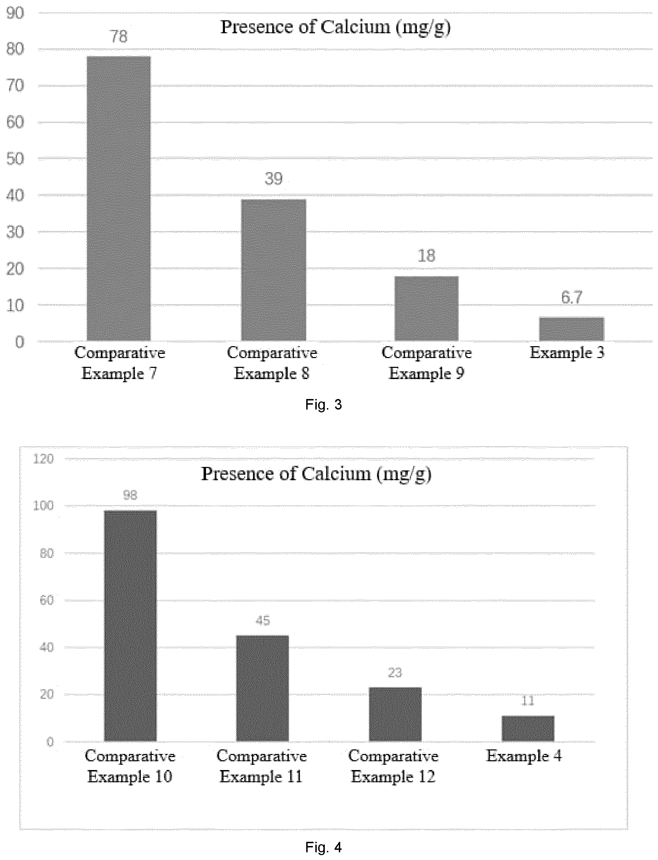

The biologically-derived materials from Example 3 and Comparative Examples 7, 8 and 9 were separately subcutaneously implanted into rats and taken out 8 weeks later. Presence of calcium on the biologically-derived materials was then measured using atomic absorption spectrophotometry, and the results are presented in Fig. 3. As can be seen from Fig. 3, the biologically-derived material obtained without end-capping (i.e., the biologically-derived material from Comparative Example 7) showed the greatest presence of calcium, and the biologically-derived material obtained by subjecting the tissue to only the first end-capping process (i.e., the biologically-derived material from Comparative Example 8) showed the second greatest presence of calcium. Moreover, the biologically-derived material obtained by subjecting the tissue to only the first and second end-capping processes (i.e., the biologically-derived material from Comparative Example 9) showed the third greatest presence of calcium, and the biologically-derived material obtained by subjecting the tissue to all the three end-capping processes and the anti-oxidation and sterilization process (i.e., the biologically-derived material from Example 3) showed the least presence of calcium, i.e., the biologically-derived material obtained by subjecting the tissue to all the three end-capping processes and the anti-oxidation and sterilization process has the best resistance to calcification.

### <Example 4>

In Example 4, a bovine pericardium was treated as biological tissue as follows.

First of all, the bovine pericardium was freshly obtained from a slaughterhouse. After undergoing stripping, selection and cleaning processes, it was subjected to a cross-linking process carried out for 14 days using a glutaraldehyde solution containing glutaraldehyde at a weight percentage of 2%. The bovine pericardium from the cross-linking process was then cut (on a cutter, model Trotec Speed x-100) into a 50 mm × 50 mm sample.

After that, a first cleaning process was performed on the sample at room temperature using a phosphate buffer solution with pH of 7.4. In this process, the sample was cleaned five times, 5 min each time.

Next, the sample from the first cleaning process was subjected to a first end-capping process carried out at 50 °C for 2 h using a first end-capping solution. The first end-capping solution has a pH of 8.6 and contained galactose and formaldehyde as a first end-capping agent. The galactose was present at a weight percentage of 9.5%, and the formaldehyde was present at a weight percentage of 3.3%.

Subsequently, the sample from the first end-capping process was subjected to a second cleaning process carried out at room temperature. In the second cleaning process, a glutaraldehyde solution containing glutaraldehyde at a weight percentage of 2% and having a pH of 7.4 was used as a cleaning agent, and the sample was cleaned three times, 3 min each time.

Afterwards, the sample from the second cleaning process was subjected to a second end-capping process carried out at 37 °C for 48 h using a second end-capping solution. The second end-capping solution had a pH of 8.4 and contained a glucosamine sulfate as a second end-capping agent at a weight percentage of 8.6%.

A third cleaning process was then performed at room temperature on the sample from the second end-capping process. In the third cleaning process, the second end-capping solution was used as a cleaning agent, and the sample was cleaned five times, 5 min each time.

After that, a third end-capping solution was used to perform a third end-capping process at room temperature on the sample from the third cleaning process. The process was carried out for 72 h, and the third end-capping solution has a pH of 8.0 and contained 2-hydroxyethylamine as a third end-capping agent at a weight percentage of 0.5%.

Afterwards, the sample from the third end-capping process was subjected to a fourth cleaning process performed at room temperature using the third end-capping solution as a cleaning agent. In the process, the sample was cleaned three times, 3 min each time.

Finally, a combined solution of an antioxidant and a sterilizing agent was used to perform an anti-oxidation and sterilization process on the sample from the fourth cleaning process at 5 °C, obtaining a biologically-derived material. The anti-oxidation and sterilization process was carried out for 72 h, and the combined solution has a pH of 7.1. The antioxidant was glutaraldehyde and present at a weight percentage of 0.625%, and the sterilizing agent was vitamin E and present at a weight percentage of 0.5%.

### <Comparative Example 10>

Comparative Example 10 differs from Example 4 in that a bovine pericardium was subjected only to the cross-linking process and the first cleaning process but not to the three end-capping processes and the second, third and fourth cleaning processes.

### <Comparative Example 11>

Comparative Example 11 differs from Example 4 in that a bovine pericardium was subjected only to the cross-linking process, the first cleaning process, the first end-capping process and the second cleaning process but not to the second end-capping process, the third cleaning process, the third end-capping process, the fourth cleaning process and the anti-oxidation and sterilization process.

### <Comparative Example 12>

Comparative Example 12 differs from Example 4 in that a bovine pericardium was subjected only to the cross-linking process, the first cleaning process, the first end-capping process, the second cleaning process, the second end-capping process and the third cleaning process but not to the third end-capping process, the fourth cleaning process and the anti-oxidation and sterilization process.

The biologically-derived materials from Example 4 and Comparative Examples 10, 11 and 12 were separately subcutaneously implanted into rats and taken out 8 weeks later. Presence of calcium on them was then measured using atomic absorption spectrophotometry, and the results are presented in Fig. 4. As can be seen from Fig. 4, the biologically-derived material obtained without end-capping (i.e., the biologically-derived material from Comparative Example 10) showed the greatest presence of calcium, and the biologically-derived material obtained by subjecting the tissue to only the first end-capping process (i.e., the biologically-derived material from Comparative Example 11) showed the second greatest presence of calcium. Moreover, the biologically-derived material obtained by subjecting the tissue to only the first and second end-capping processes (i.e., the biologically-derived material from Comparative Example 12) showed the third greatest presence of calcium, and the biologically-derived material obtained by subjecting the tissue to all the three end-capping processes and the anti-oxidation and sterilization process (i.e., the biologically-derived material from Example 4) showed the least presence of calcium, i.e., the biologically-derived material obtained by subjecting the tissue to all the three end-capping processes and the anti-oxidation and sterilization process has the best resistance to calcification.

### <Example 5>

In Example 5, a bovine pericardium was treated as biological tissue as follows.

First of all, the bovine pericardium was freshly obtained from a slaughterhouse. After undergoing stripping, selection and cleaning processes, it was subjected to a cross-linking process carried out for 14 days using a glutaraldehyde solution containing glutaraldehyde at a weight percentage of 2%. The bovine pericardium from the cross-linking process was then cut (on a cutter, model Trotec Speed x-100) into a 50 mm × 50 mm sample.

After that, a first cleaning process was performed on the sample at room temperature using a phosphate buffer solution with pH of 7.4. In this process, the sample was cleaned five times, 5 min each time.

Next, the sample from the first cleaning process was subjected to a first end-capping process carried out at 50 °C for 2 h using a first end-capping solution. The first end-capping solution has a pH of 8.6 and contained galactose and formaldehyde as a first end-capping agent. The galactose was present at a weight percentage of 9.5%, and the formaldehyde was present at a weight percentage of 3.3%.

Subsequently, the sample from the first end-capping process was subjected to a second cleaning process carried out at room temperature. In the second cleaning process, a glutaraldehyde solution containing glutaraldehyde at a weight percentage of 2% and having a pH of 7.4 was used as a cleaning agent, and the sample was cleaned three times, 3 min each time.

Afterwards, the sample from the second cleaning process was subjected to a second end-capping process carried out at 10 °C for 72 h using a second end-capping solution. The second end-capping solution had a pH of 7.4 and contained a glucosamine sulfate and sodium borohydride as a second end-capping agent. The glucosamine sulfate was present at a weight percentage of 8.6%, and the sodium borohydride was present at a weight percentage of 2.1%.

A third cleaning process was then performed at room temperature on the sample from the second end-capping process. In the third cleaning process, the second end-capping solution was used as a cleaning agent, and the sample was cleaned five times, 5 min each time.

After that, a third end-capping solution was used to perform a third end-capping process at room temperature on the sample from the third cleaning process. The process was carried out for 72 h, and the third end-capping solution has a pH of 8.0 and contained 2-hydroxyethylamine as a third end-capping agent at a weight percentage of 0.5%.

Afterwards, the sample from the third end-capping process was subjected to a fourth cleaning process performed at room temperature using the third end-capping solution as a cleaning agent. In the process, the sample was cleaned three times, 3 min each time.

Finally, a combined solution of an antioxidant and a sterilizing agent was used to perform an anti-oxidation and sterilization process on the sample from the fourth cleaning process at 5 °C, obtaining a biologically-derived material. The anti-oxidation and sterilization process was carried out for 72 h, and the combined solution has a pH of 6.8. The sterilizing agent was glutaraldehyde and present at a weight percentage of 0.625%, and the antioxidant was vitamin E and present at a weight percentage of 2.3%.

### <Comparative Example 13>

Comparative Example 13 differs from Example 5 in that a bovine pericardium was subjected only to the cross-linking process and the first cleaning process but not to the three end-capping processes and the second, third and fourth cleaning processes.

### <Comparative Example 14>

Comparative Example 14 differs from Example 5 in that a bovine pericardium was subjected only to the cross-linking process, the first cleaning process, the first end-capping process and the second cleaning process but not to the second end-capping process, the third cleaning process, the third end-capping process, the fourth cleaning process and the anti-oxidation and sterilization process.

### <Comparative Example 15>

Comparative Example 15 differs from Example 5 in that a bovine pericardium was subjected only to the cross-linking process, the first cleaning process, the first end-capping process, the second cleaning process, the second end-capping process and the third cleaning process but not to the third end-capping process, the fourth cleaning process and the anti-oxidation and sterilization process.

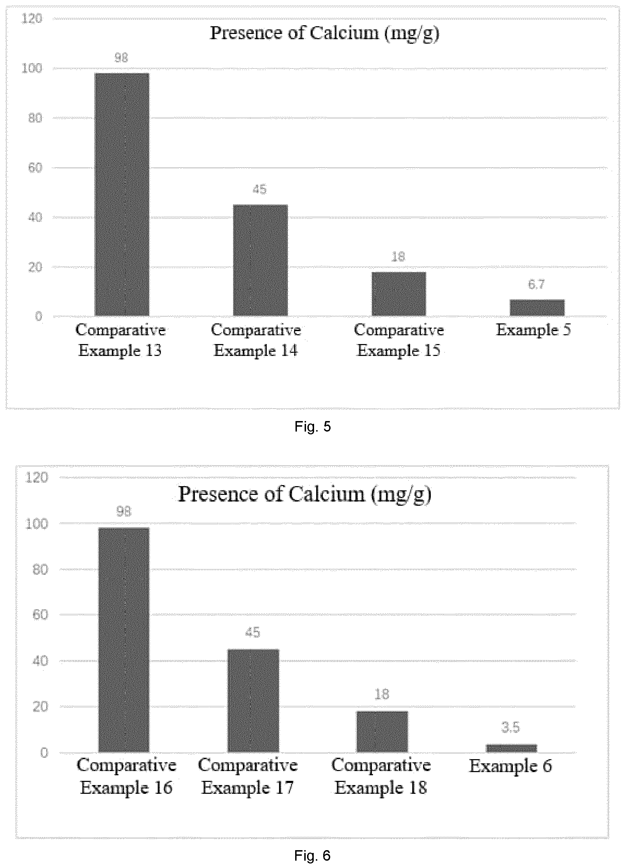

The biologically-derived materials from Example 5 and Comparative Examples 13, 14 and 15 were separately subcutaneously implanted into rats and taken out 8 weeks later. Presence of calcium on them was then measured using atomic absorption spectrophotometry, and the results are presented in Fig. 5. As can be seen from Fig. 5, the biologically-derived material obtained without end-capping (i.e., the biologically-derived material from Comparative Example 13) showed the greatest presence of calcium, and the biologically-derived material obtained by subjecting the tissue to only the first end-capping process (i.e., the biologically-derived material from Comparative Example 14) showed the second greatest presence of calcium. Moreover, the biologically-derived material obtained by subjecting the tissue to only the first and second end-capping processes (i.e., the biologically-derived material from Comparative Example 15) showed the third greatest presence of calcium, and the biologically-derived material obtained by subjecting the tissue to all the three end-capping processes and the anti-oxidation and sterilization process (i.e., the biologically-derived material from Example 5) showed the least presence of calcium, i.e., the biologically-derived material obtained by subjecting the tissue to all the three end-capping processes and the anti-oxidation and sterilization process has the best resistance to calcification.

### <Example 6>

In Example 6, a bovine pericardium was treated as biological tissue as follows.

First of all, the bovine pericardium was freshly obtained from a slaughterhouse. After undergoing stripping, selection and cleaning processes, it was subjected to a cross-linking process carried out for 14 days using a glutaraldehyde solution containing glutaraldehyde at a weight percentage of 2%. The bovine pericardium from the cross-linking process was then cut (on a cutter, model Trotec Speed x-100) into a 50 mm × 50 mm sample.

After that, a first cleaning process was performed on the sample at room temperature using a phosphate buffer solution with pH of 7.4. In this process, the sample was cleaned five times, 5 min each time.

Next, the sample from the first cleaning process was subjected to a first end-capping process carried out at 50 °C for 2 h using a first end-capping solution. The first end-capping solution has a pH of 8.6 and contained galactose and formaldehyde as a first end-capping agent. The galactose was present at a weight percentage of 9.5%, and the formaldehyde was present at a weight percentage of 3.3%.

Subsequently, the sample from the first end-capping process was subjected to a second cleaning process carried out at room temperature. In the second cleaning process, a glutaraldehyde solution containing glutaraldehyde at a weight percentage of 2% and having a pH of 7.4 was used as a cleaning agent, and the sample was cleaned three times, 3 min each time.

Afterwards, the sample from the second cleaning process was subjected to a second end-capping process carried out at 37 °C for 48 h using a second end-capping solution. The second end-capping solution had a pH of 8.4 and contained a glucosamine sulfate and sodium borohydride as a second end-capping agent. The glucosamine sulfate was present at a weight percentage of 8.6%, and the sodium borohydride was present at a weight percentage of 2.1%.

A third cleaning process was then performed at room temperature on the sample from the second end-capping process. In the third cleaning process, the second end-capping solution was used as a cleaning agent, and the sample was cleaned five times, 5 min each time.

After that, a third end-capping solution was used to perform a third end-capping process at room temperature on the sample from the third cleaning process. The process was carried out for 72 h, and the third end-capping solution has a pH of 8.0 and contained 2-hydroxyethylamine and EDC as a third end-capping agent. The 2-hydroxyethylamine was present at a weight percentage of 0.5%, and the EDC was present at a weight percentage of 7.3%.

Afterwards, the sample from the third end-capping process was subjected to a fourth cleaning process performed at room temperature using the third end-capping solution as a cleaning agent. In the process, the sample was cleaned three times, 3 min each time.

Finally, a combined solution of an antioxidant and a sterilizing agent was used to perform an anti-oxidation and sterilization process on the sample from the fourth cleaning process at 5 °C, obtaining a biologically-derived material. The anti-oxidation and sterilization process was carried out for 72 h, and the combined solution has a pH of 7.4. The sterilizing agent was glutaraldehyde and present at a weight percentage of 0.625%, and the antioxidant was vitamin E and present at a weight percentage of 0.5%.

### <Comparative Example 16>

Comparative Example 16 differs from Example 6 in that a bovine pericardium was subjected only to the cross-linking process and the first cleaning process but not to the three end-capping processes and the second, third and fourth cleaning processes.

### <Comparative Example 17>

Comparative Example 17 differs from Example 6 in that a bovine pericardium was subjected only to the cross-linking process, the first cleaning process, the first end-capping process and the second cleaning process but not to the second end-capping process, the third cleaning process, the third end-capping process, the fourth cleaning process and the anti-oxidation and sterilization process.

### <Comparative Example 18>

Comparative Example 18 differs from Example 6 in that a bovine pericardium was subjected only to the cross-linking process, the first cleaning process, the first end-capping process, the second cleaning process, the second end-capping process and the third cleaning process but not to the third end-capping process, the fourth cleaning process and the anti-oxidation and sterilization process.

The biologically-derived materials from Example 6 and Comparative Examples 16, 17 and 18 were separately subcutaneously implanted into rats and taken out 8 weeks later. Presence of calcium on them was then measured using atomic absorption spectrophotometry, and the results are presented in Fig. 6. As can be seen from Fig. 6, the biologically-derived material obtained without end-capping (i.e., the biologically-derived material from Comparative Example 16) showed the greatest presence of calcium, and the biologically-derived material obtained by subjecting the tissue to only the first end-capping process (i.e., the biologically-derived material from Comparative Example 17) showed the second greatest presence of calcium. Moreover, the biologically-derived material obtained by subjecting the tissue to only the first and second end-capping processes (i.e., the biologically-derived material from Comparative Example 18) showed the third greatest presence of calcium, and the biologically-derived material obtained by subjecting the tissue to all the three end-capping processes and the anti-oxidation and sterilization process (i.e., the biologically-derived material from Example 6) showed the least presence of calcium, i.e., the biologically-derived material obtained by subjecting the tissue to all the three end-capping processes and the anti-oxidation and sterilization process has the best resistance to calcification.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A method of treating biological tissue, comprising the steps of:
performing a cross-linking process on the biological tissue, wherein a surface of the biological tissue subjected to the cross-linking process has active groups;
performing an end-capping process on the biological tissue subjected to the cross-linking process so that at least some of the active groups are end-capped; and
performing an anti-oxidation and sterilization process on the biological tissue subjected to the end-capping process.

2. The method of treating biological tissue of claim 1, wherein a cross-linking agent used comprises glutaraldehyde, wherein the active groups include amino groups, free aldehyde groups and free carboxyl groups.

3. The method of treating biological tissue of claim 1 or 2, further comprising:
after the biological tissue is subjected to the cross-linking process and before the biological tissue from the cross-linking process is subjected to the end-capping process,
performing a first cleaning process on the biological tissue.

4. The method of treating biological tissue of claim 3, wherein a cleaning agent used in the first cleaning process comprises any of a physiological saline solution, a phosphate buffer with a pH of 6.8 to 8.6, a D-Hanks solution with a pH of 6.8 to 8.6 or a glutaraldehyde solution with a weight percentage of glutaraldehyde of 0.1% to 2% and a pH of 6.8 to 8.6.

5. The method of treating biological tissue of claim 2, wherein the end-capping process comprises:
subjecting the biological tissue from the cross-linking process to a first end-capping step using a first end-capping solution to end-cap at least some of the amino groups;
subjecting the biological tissue from the first end-capping step to a second end-capping step using a second end-capping solution to end-cap at least some of the free aldehyde groups; and
performing subjecting the biological tissue from the second end-capping step to a third end-capping step using a third end-capping solution to end-cap at least some of the free carboxyl groups.

6. The method of treating biological tissue of claim 5, wherein the first end-capping solution comprises a first end-capping agent comprising at least one of heparin, mannose, formaldehyde and galactose, and/or
wherein the second end-capping solution comprises a second end-capping agent comprising at least one of sodium borohydride, taurine, polyvinyl alcohol and an aminosulfate, and/or
wherein the third end-capping solution comprises a third end-capping agent comprising at least one of N-hydroxysulfosuccinimide, 1-(3-dimethylaminopropyl)-3-ethylcarbodiimide hydrochloride and an amino compound.

7. The method of treating biological tissue of claim 6, wherein the first end-capping solution has a pH of 5.0 to 9.0, with any component of the first end-capping agent being present in the first end-capping solution at a weight percentage of 0.01% to 10% and the first end-capping step being performed at a temperature of 0 to 50 °C for 1 h to 72 h,
wherein the second end-capping solution has a pH of 5.0 to 9.0, with any component of the second end-capping agent being present in the second end-capping solution at a weight percentage of 0.01% to 10% and the second end-capping step being performed at a temperature of 0 to 50 °C for 1 h to 72 h, and
wherein the third end-capping solution has a pH of 5.0 to 9.0, with any component of the third end-capping agent being present in the third end-capping solution at a weight percentage of 0.01% to 10% and the third end-capping step being performed at a temperature of 0 to 50 °C for 1 h to 72 h.

8. The method of treating biological tissue of claim 5, wherein after the first end-capping step and prior to the second end-capping step, the end-capping process further comprises subjecting the biological tissue from the first end-capping step to a second cleaning process;
wherein after the second end-capping step and prior to the third end-capping step, the end-capping process further comprises subjecting the biological tissue from the second end-capping step to a third cleaning process; and
wherein after the third end-capping step and before the biological tissue from the end-capping process is subjected to the anti-oxidation and sterilization process, the end-capping process further comprises subjecting the biological tissue to a fourth cleaning process.

9. The method of treating biological tissue of claim 8, wherein the second cleaning process uses a glutaraldehyde solution as a cleaning agent, which contains glutaraldehyde at a weight percentage of 0.01% to 2% and has a pH of 6.8 to 8.6, and/or
wherein the third cleaning process uses the second end-capping agent as a cleaning agent, and/or
wherein the fourth cleaning process uses the third end-capping agent as a cleaning agent.

10. The method of treating biological tissue of claim 1, wherein the anti-oxidation and sterilization process is performed on the biological tissue from the end-capping process using a combined solution of a sterilizing agent and an antioxidant, the sterilizing agent comprising any of glutaraldehyde and formaldehyde, the antioxidant comprising at least one of vitamin C, vitamin E and a reducing sugar, the antioxidant present in the combined solution at a weight percentage of 0.01% to 10%, the sterilizing agent present in the combined solution at a weight percentage of 0.01% to 10%, the combined solution having a pH of 6.8 to 8.6.

11. The method of treating biological tissue of claim 10, wherein the anti-oxidation and sterilization process is performed at a temperature of 0 to 50 °C for 1 h to 72 h.

12. The method of treating biological tissue of claim 1, wherein the biological tissue comprises any of a pericardium, heart valve, pulmonary pleuron, small intestinal submucosa, cranial dura mater, spinal dura mater, ligament and animal skin.

13. A medical material, being obtained by treating biological tissue according to the method of treating biological tissue of any one of claims 1 to 12.
